# EUROPEAN PATENT APPLICATION

(11) **EP 3 715 471 A1**
(43) Date of publication of application: **30.09.2020**
(21) Application number: 19166374.9
(22) Date of filing: 29.03.2019
(51) Int. Cl.: C12Q 1/6876

(54) **AHR SIGNATURE MARKER SET**

(71) Applicant: Deutsches Krebsforschungszentrum, Stiftung des öffentlichen Rechts, 69120 Heidelberg (DE); Helmholtz-Zentrum für Umweltforschung GmbH - UFZ, 04318 Leipzig (DE); Friedrich-Schiller-Universität Jena (FSU), 07743 Jena (DE)
(72) Inventor: Optiz, Christiane, 69120 Heidelberg (DE); Sadik, Ahmed, 69120 Heidelberg (DE); Somarribas Patterson, Luis Felipe, 69120 Heidelberg (DE); Mohapatra, Soumya, 69120 Heidelberg (DE); Trump, Saskia, 04105 Leipzig (DE); Schäuble, Sacha, 07749 Jena (DE); Fäßler, Erik, 07745 Jena (DE)
(74) Representative: Krauss, Jan

(57) **Abstract**

The present invention relates to the generation and uses of an improved set of genetic markers that are aryl hydrocarbon receptor (AHR) target genes, designated as "AHR signature". This new signature allows to efficiently determine AHR activation subgroups, for example for an improved classification of tumors. The signature comprises markers that are important in diagnosis and therapy, for example for selecting patients for treatment with AHR activation modulating interventions, and monitoring of therapy response.

## Description

The present invention relates to the generation and uses of an improved set of genetic markers that are aryl hydrocarbon receptor (AHR) target genes, designated as "AHR signature". This new AHR signature allows to efficiently determine AHR activation sub-groups, in particular for an improved classification of tumors. The signature comprises markers that are important in diagnosis and therapy, for example for selecting patients for treatment with AHR activation modulating interventions, and monitoring of therapy response.

### Background of the invention

The aryl hydrocarbon receptor (AHR) is a ligand-activated transcription factor involved in the regulation of diverse processes such as embryogenesis, vasculogenesis, drug metabolism, cell motility and immune modulation, and cancer. In preclinical studies AHR activation by tryptophan metabolites generated through indoleamine-2,3-dioxygenase (IDO1) and/or tryptophan-2,3-dioxygenase (TDO2) promoted tumor progression by enhancing the motility, anoikis and clonogenic survival of the tumor cells as well as by suppressing anti-tumor immune responses.

As ligand binding is necessary for AHR activation, the expression level of AHR alone does not allow inference of its activation state. AHR activation is commonly detected by its nuclear translocation, the activity of cytochrome P-450 enzymes or the binding of AHR-ARNT to dioxin-responsive elements (DRE) using reporter assays. While all of these methods are applicable *in vitro,* they are laborious, require special equipment and are expensive. In addition, relying on cytochrome P-450 enzymes is limited to conditions where they are expressed, which is not always the case, given the ligand and cell type specificity of AHR activation. Several attempts to study the activation of AHR are known from the literature.

Vezina et al. (in: Subchronic exposure to TCDD, PeCDF, PCB126, and PCB153: effect on hepatic gene expression. Environ Health Perspect. 2004; 112(16):1636-44) studied the AHR ligands TCDD, PeCDF, and PCB126, which produced very similar global gene expression profiles, underscoring the extensive impact of AhR activation and/or the resulting hepatic injury on global gene expression in female rat liver. They only studied some of the fundamental features of dioxin toxicity to further clarify the biologic role of the AhR signaling pathway.

Vacher et al. (in: High AHR expression in breast tumors correlates with expression of genes from several signaling pathways namely inflammation and endogenous tryptophan metabolism. PLoS One. 2018;13(1):e0190619) identified breast tumors (ERα-positive or ERα-negative) that express *AHR* and how AhR affects human tumorigenesis. The levels of *AHR, AHR* nuclear translocator (*ARNT*) and *AHR* repressor (*AHRR*) mRNA expression were analyzed in a cohort of 439 breast tumors, High *AHR* expression was correlated with high expression of several genes involved in signaling pathways related to inflammation (*IL1B, IL6, TNF, IL8* and *CXCR4*), metabolism (*IDO1* and *TDO2* from the kynurenine pathway), invasion (*MMP1, MMP2* and *PLAU*), and IGF signaling (*IGF2R, IGF1R* and *TGFB1*). Two ligands for *AHR* (TCDD and BaP) induced mRNA expression of *IL1B* and *IL6* in an ERα-negative breast tumor cell line.

Guastella et al. (in: Investigation of the aryl hydrocarbon receptor and the intrinsic tumoral component of the kynurenine pathway of tryptophan metabolism in primary brain tumors. J Neurooncol. 2018 Sep;139(2):239-249) studied brain tumors, and suggested that AhR may offer a novel and robust therapeutic target for a patient population with highly limited treatment options.

Wu et al. (in: A novel integrated gene coexpression analysis approach reveals a prognostic three-transcription-factor signature for glioma molecular subtypes. BMC Syst Biol. 2016 Aug 26;10 Suppl 3:71) designed a novel integrated gene coexpression analysis approach, which involves differential coexpression and differential regulation analysis (DCEA and DRA), to investigate glioma prognostic biomarkers and molecular subtypes based on six glioma transcriptome data sets. They proposed a three-transcription-factor signature including AHR, NFIL3 and ZNF423 for glioma molecular subtypes. This three-TF signature clusters glioma patients into three major subtypes (ZG, NG and IG subtypes) which are significantly different in patient survival as well as transcriptomic patterns. Nevertheless, in the heatmaps most of the groups show a somewhat similar expression gradient of AHR and NFIL3.

WO 2013/171696 discloses a method for sorting substances for the purpose of determining their capacity for sebosuppressive activity in topical skin treatment, comprising an in vivo test comprising choosing a substance from among the ligands of the AhR receptor. The application relies on rodent Cyp1A1.

JP2008228627 discloses an AhR (allyl hydrocarbon receptor) chimeric protein useful for highly sensitively detecting the toxicity of chemical substances. Similarly, US2004043394 discloses a gene expression element specific for AHR ligands and heterologous gene expression systems dependent on the element.

AHR target gene expression is context-specific, and therefore an AHR activation signature consisting of diverse AHR target genes is required to efficiently detect AHR activation across different cells/tissues and in response to diverse AHR ligands. It is therefore an object of the present invention, to provide a transcriptional AHR activation signature that enables a reliable detection of AHR activation in human tissues, while maintaining sufficient complexity. Furthermore, additional genes are sought after as markers that help to further understand the complex functions of AHR in particular the context of diseases and conditions related with AHR.

In a first aspect thereof, the present invention solves this problem by providing a panel of biomarkers (also called "markers" or "genes" herein), comprising about 5, about 10, about 20, about 30 of said AHR biomarkers according to table 1 or at least 10%, at least 20%, at least 30%, at least 40%, at least 50%, at least 60%, at least 70%, at least 80% or at least 90% or more or all of the genes according to table 1.

In a second aspect thereof, the present invention relates to a method for determining an AHR activation signature for a biological sample, comprising i) detecting at least one biological state of at least one AHR biomarker according to table 1 for said sample, ii) identifying a change of said biological state of said at least one AHR biomarker compared to a house keeping gene or control biomarker, and iii) assigning said at least one AHR biomarker to said AHR activation signature for said biological sample, if a) said at least one biomarker provides a significance of said AHR activation signature of p < 0.05 at a minimal number of markers in the signature and/or b) a fold of change of said AHR activation signature of at least about 1.5 at a minimal number of markers in the signature in the case of up-regulation or of at least about 0.67 at a minimal number of markers in the signature in the case of down-regulation. The method can be in vivo or in vitro, including that the exposure of the cells/samples to AHR modulators could be from external sources, applied directly to the cells or as a result of an endogenous modulator that affects AHR activation both directly or indirectly.

In a third aspect thereof, the present invention relates to a method for monitoring AHR activation in a biological sample in response to at least one compound, comprising performing the method according to the present invention on samples that have been obtained during the course of contacting said sample with at least one modulator (also sometimes referred to as "compound" or "modulator compound" herein). The modulator compound can be directly applied to the sample in vitro or through different routes of administration for example parentral preparations, ingestion, topical application, vaccines or others.

In a fourth aspect thereof, the present invention relates to a method for screening for a modulator compound of AHR activation genes, comprising performing the method according to the present invention, and further comprising contacting at least one candidate modulator compound with said biological sample, wherein a change in the biological state of said at least one AHR biomarker of said signature in the presence of said at least one compound compared to the absence of said at least compound identifies a modulator. The modulator compound of AHR activation genes can modulate said genes directly or indirectly, i.e. by acting on AHR directly or indirectly by acting on a signaling pathway upstream of the AHR marker.

In a fifth aspect thereof, the present invention relates to a method for testing the biological safety of a compound, comprising performing a method according to the present invention, and further comprising the step of concluding on the safety of said compound based on said effect as identified.

Another important aspect of the present invention relates to a diagnostic kit comprising materials for performing a method according to the present invention in one or separate containers, optionally together with auxiliary agents and/or instructions for performing said method.

Finally, the invention relates to the use of at least one biomarker or a panel of biomarkers of about 5, about 10, about 20, about 30 of said AHR biomarkers according to table 1 or at least 10%, at least 20%, at least 30%, at least 40%, at least 50%, at least 60%, at least 70%, at least 80% or at least 90% or more of the genes according to table 1 for monitoring AHR activation in a biological sample according to the present invention, or for screening for a modulator of AHR activation genes according to the present invention, or for testing the biological safety according to the present invention or for a diagnosis according to the present invention.

Other aspects and advantages can be readily derived from reading the following description and the non-limiting examples.

As mentioned above, in the context of the experiments as performed in the context of the present invention, an improved panel of AHR-biomarkers was identified, comprising about 5, about 10, about 20, about 30 of said AHR biomarkers according to table 1 or at least 10%, at least 20%, at least 30%, at least 40%, at least 50%, at least 60%, at least 70%, at least 80% or at least 90% or more or all of said AHR biomarkers according to table 1.

Using this panel, a multitude of methods with respect to functional studies of AHR become enabled. In one aspect thereof, the present invention relates to an in-vitro method for screening for a modulator of the expression of AHR-regulated genes, comprising contacting a cell with at least one candidate modulator compound, and detecting at least one of mutations, nucleic acid methylation, copy numbers, expression, amount of protein, metabolites and activity of said genes of the AHR-signature according to table 1, wherein a change as detected of about 5, about 10, about 20, about 30 of said AHR biomarkers according to table 1 or at least 10%, at least 20%, at least 30%, at least 40%, at least 50%, at least 60%, at least 70%, at least 80% or at least 90% or more or all of said AHR biomarkers according to table 1 in the presence of said at least one compound compared to the absence of said at least compound identifies a modulator. This modulator in preferred embodiments can be used in additional steps of the method where a classifier is used or activation is evaluated based on the signature compared to house keeping genes or control biomarkers as disclosed herein.

The main obstacle in order to gain an in-depth understanding of the roles and functions of AHR is the complexity of the analysis, which in current approaches applied, there is a considerable loss of information that led to unsatisfying and insufficient results in the examination of AHR activation or function. As ligand binding is necessary for AHR activation, the expression level of AHR alone does not allow inference of its activation state. AHR activation is commonly detected by its nuclear translocation, the activity of cytochrome P-450 enzymes or the binding of AHR-ARNT to dioxin-responsive elements (DRE) using reporter assays. While all of these methods are applicable in vitro, they are laborious, require special equipment and are expensive. In addition, relying on cytochrome P-450 enzymes is limited to conditions where they are expressed, which is not always the case, given the ligand and cell type specificity of AHR activation. AHR target gene expression is context-specific, implying that an AHR activation signature consisting of diverse AHR target genes is required to detect AHR activation across different cells/tissues and in response to diverse AHR ligands. The inventors therefore set out to develop a transcriptional AHR activation signature to enable detection of AHR activation in different tissues/cell types. Generally preferred are the human samples and human variants of the biomarkers, or closely related species, like other primates or mammals.

Therefore, here the inventors developed a transcriptional AHR activation signature employing expression dataset analysis combined with natural language processing to investigate AHR activation in human cancer tissues. IDO1 and TDO2 expression positively associated with AHR activation in multiple tumor entities. The signature was also grouped into eight biological functional groups represented by gene ontology terms that are enriched in the AHR signature genes. The main functions known to be modulated by AHR activation include drug metabolism, cell motility and immune modulation. The AHR signature as established can detect AHR activation across different cell/tissue types and in response to diverse ligands. Using the AHR signature, it is possible to determine AHR activation sub-groups by unsupervised clustering methods, which can be utilized for classification of samples. This is important for example, in terms of selecting patients for treatment with AHR activation modulating interventions, and monitoring of therapy response.

In a preferred aspect thereof, the present invention relates to a method for determining an AHR activation signature for a biological sample, comprising detecting at least one biological state of at least one AHR biomarker according to table 1 for said sample, identifying a change of said biological state of said at least one AHR biomarker compared to a house keeping gene or control biomarker, and assigning said at least one AHR biomarker to said AHR activation signature for said biological sample, if said at least one biomarker provides a significance of said AHR activation signature of p < 0.05 at a minimal number of markers in the signature and/or an absolute fold of change of said AHR activation signature of at least about 1.5 at a minimal number of markers in the signature in the case of up-regulation or at least about 0.67 at a minimal number of markers in the signature in the case of down regulation. Therefore, in this context, the AHR activation signature according to the present invention is used as an improved resource for refined sub-signatures or context specific signatures that are tailor-made for specific use in the context of different biological processes in samples to be examined that involve the detection or evaluation of AHR activation and its functional implications.

In the first step of the method, a sample to be examined is provided. In the context of the present invention, any biological sample can be used as long as it contains (or is presumed to contain) at least one of the biomarker(s) to be used in the analysis and/or screen. Preferably, the biological sample is selected from a sample comprising biological fluids comprising biomarkers, cells, tissues, whole blood, cell lines, primary cells, IPCs, hybridomas, human cells, recombinant cells, stem cells, cancer cells, bone cells, cartilage cells, nerve cells, glial cells, epithelial cells, skin cells, scalp cells, lung cells, mucosal cells, muscle cells, skeletal muscles cells, straited muscle cells, smooth muscle cells, heart cells, secretory cells, adipose cells, blood cells, erythrocytes, basophils, eosinophils, monocytes, lymphocytes, T-cells, B-cells, neutrophils, NK cells, regulatory T-cells, dendritic cells, Th17 cells, Th1 cells, Th2 cells, myeloid cells, macrophages, monocyte derived stromal cells, bone marrow cells, spleen cells, thymus cells, pancreatic cells, oocytes, sperm, kidney cells, fibroblasts, intestinal cells, cells of the female or male reproductive tracts, prostate cells, bladder cells, eye cells, corneal cells, retinal cells, sensory cells, keratinocytes, hepatic cells, brain cells, kidney cells, and colon cells, and the transformed counterparts of said cell types. The sample can also be selected from tumor tissue (tumor or metastases), biopsies, whole blood, peripheral blood, or fractions thereof, serum, buffy coat, lymphatic fluid, urine, bone marrow, heparinized whole blood, and frozen samples thereof, such as frozen heparinized whole blood or tissues, and formalin fixed paraffin embedded tissues. The cells to be used in the methods according to the present invention can be genetically modified depending on the desired purpose and circumstances. Totipotent human embryonic stem cells may be excluded, if necessary.

In the next step, a change of a biological state of said at least one AHR biomarker is identified, compared to at least one house keeping gene and/or at least one suitable control biomarker. As preferred examples, said biological state as detected is selected from mutations, nucleic acid methylation, copy numbers, expression, amount of protein, metabolite, and activity of said at least one AHR biomarker.

In the context of the present invention, a housekeeping gene shall refer to a constitutive gene that is expressed in all cells of the biological sample to be analysed. Usually housekeeping genes are selected by the person of skill based on their requirement for the maintenance of basic cellular function in the cells of the sample as analysed under normal, and patho-physiological conditions (if present in the context of the analysis). Examples of housekeeping genes are known to the person of skill, and may involve the ones as disclosed, e.g. in Eisenberg E, Levanon EY (October 2013). "Human housekeeping genes, revisited". Trends in Genetics. 29 (10): 569-574.

A preferred aspect of the method according to the present invention, said method further involves the step of identifying at least one suitable house keeping gene and/or at least one suitable control biomarker for the sample to be analysed, comprising detecting the expression and/or biological function of a potentially suitable house keeping gene and/or control biomarker in said sample, and identifying said house keeping gene and/or control biomarker as suitable, if said expression and/or biological function does not change or substantially change over time, when compared to the markers of the respective AHR signature as analysed (control biomarker). Another suitable marker is the non-mutated version of a marker of the respective AHR signature as analysed. Therefore, control biomarkers can be markers independent from the AHR signature or be part of the signature itself (particularly in case of mutations).

In the context of the present invention, the at least one AHR biomarker is then assigned to said AHR activation signature for said biological sample. For this, in one embodiment the marker must show an absolute fold of change of said AHR activation signature of at least about 1.5 at a minimal number of markers in the signature in the case of up-regulation or f at least about 0.67 at a minimal number of markers in the signature in the case of down-regulation. Thus, a panel is created that contains as few as possible markers (i.e. 1, 2, 3, etc.) based on the most "prominent" changes as identified. This embodiment is particularly useful in cases where only a few markers are selected, e.g. in the context of a kit of markers and/or a point of care test, without the necessity of substantial machinery and equipment. Preferred is a method according to the present invention, wherein said absolute fold of change of said AHR activation signature is at least about 1.5, preferably at least about 1.8, more preferably at least about 2, and more preferably at least about 3 or more in the case of up-regulation, or wherein said absolute fold of change of said AHR activation signature is at least about 0.67, preferably at least about 0.57, more preferably at least about 0.25 or more in the case of down regulation.

Preferred is a method according to the present invention, wherein said AHR activation signature comprises about 5, about 10, about 20, about 30 of said AHR biomarkers according to table 1 or at least 10, at least 20%, at least 30%, at least 40%, at least 50%, at least 60%, at least 70%, at least 80% or at least 90% or more or all of said AHR biomarkers according to table 1.

In a second embodiment, the at least one biomarker to the modulation must provide a significance of said AHR activation signature of p < 0.05 at a minimal number of markers in the signature. This embodiment is particularly useful in cases where so called "high-throughput" technology is used where a number of markers are analysed in parallel, e.g. on a chip. Usually, this involves respective automation, machinery and equipment, such as a robot. Preferred is a method according to the present invention, wherein said AHR activation signature provides a significance of about p < 0.01, preferably of about p < 0.001, and more preferably of about p < 0.0001.

The above first and second embodiments can be combined, if desired, i.e. both requirements can be met, where useful and/or required.

In the context of the present invention, the term "about" shall mean to include +/- 20%, preferably +/- 10% of the amount as given, unless otherwise noted.

Preferred is a method according to the present invention, wherein said AHR activation signature is identified in a sample under physiological conditions or under disease conditions. Because of the complexity and wide-spread involvement of AHR in cellular processes the diseases and function can be extensive, and an AHR-related disease or condition can be selected from at least one of proliferative diseases, cancer, autoimmune disorders and diseases, degeneration, inflammation, infection and intoxication, or an AHR-related function or effect can be selected from at least one of proliferation, toxicity, biological safety screenings, cellular damage or stress, motility, apoptosis, stress conditions, for example, biological, mechanical and environmental stresses and senescence.

Preferred is a method according to the present invention, wherein said method further comprises the step of using said AHR activation signature for unsupervised clustering or supervised classification of said samples into AHR activation subgroups. Respective methods are known to the person of skill for example K-means clustering, hierarchical clustering, principle component analysis and non-negative matrix factorization. Clustering of the biomarkers will depend on the sample and the circumstances to be analysed, and may be based on the biological function of the biomarkers, and/or the respective functional subgroup of the AHR signature or other groups of interest, e.g. the signaling pathway or network. The AHR signature as established is also capable of detecting AHR activation across different cell/tissue types and in response to diverse ligands. Using the AHR signature, it is possible to determine AHR activation sub-groups by unsupervised clustering methods, which can be utilized for classification of samples. This is important for example, in terms of selecting patients for treatment with AHR activation modulating interventions, and monitoring of therapy response.

Preferred is a method according to the present invention, wherein said method further comprises the step of using said AHR activation signature or AHR activation subgroups to define modulated AHR activation functions, such as, for example, angiogenesis, drug metabolism, external stress response, hemopoiesis, lipid metabolism, cell motility, and immune modulation. Obviously, these functions also depend on the markers and their respective signaling pathways or networks that are modulated.

Another important aspect of the present invention then relates to a method for monitoring AHR activation in a biological sample in response to at least one modulator compound, comprising performing the method according to the present invention on biological samples/samples that have been obtained during the course of contacting said sample with at least one modulator. The modulator compound can be directly applied to the sample in vitro or through different routes of administration for example parentral preparations, ingestion, topical application, vaccines, i.v, or others, wherein a change in the AHR activation in the presence of said at least one compound compared to the absence of said at least compound indicates an effect of said at least one compound on said AHR activation. This modulator in preferred embodiments can be used in additional steps of the method where a classifier is used, or activation is evaluated based on the signature compared to house keeping genes or control biomarkers as disclosed herein..

The uses of the AHR-signature also include a method for monitoring an AHR-related disease or condition or function or effect in a cell, comprising performing a method according to the present invention, providing at least one modulator compound to said cell and detecting the change in at least one biological state of the genes of the AHR-signature in said cell in response to said at least one compound, wherein a change in the at least one biological state of the genes of said signature in the presence of said at least one compound compared to the absence of said at least compound indicates an effect of said at least one compound on said AHR-related disease or condition or function or effect.

Because of the complexity and wide-spread involvement of AHR in cellular processes the diseases and function can be quite a few, and as an AHR-related disease or condition can be selected from at least one of proliferative diseases, cancer, autoimmune diseases, and intoxication, or as an AHR-related function or effect can be selected from at least one of proliferation, toxicity, cellular damage or stress, motility, apoptosis, and senescence.

Another important aspect of the present invention then relates to a method for screening for a modulator of AHR activation genes, comprising performing the method according to the present invention, and further comprising contacting at least one candidate modulator compound with said biological sample or modulating the levels of at least one candidate modulator with said biological sample, wherein a change in the biological state of said at least one AHR biomarker of said signature in the presence of said at least one compound compared to the absence of said at least compound identifies a modulator. Preferred is such a method according to the present invention, wherein said modulator is selected from an inhibitor or an agonist of said expression or biological activity.

Therefore, methods according to the present invention are provided that in one aspect seek modulators and elucidate effects thereof on the AHR signature as identified. Some of these compounds are known, but many are still to be identified. Preferred is a method according to the present invention, wherein said modulator is selected from an inhibitor or an agonist of said expression, for example selected from TCDD, FICZ, Kyn, CH223191, SRI, a proteinaceous AHR binding domain, a small molecule, a peptide, a mutated version of a protein, for example an intracellular or recombinantly introduced protein, and a library of said compounds. Nevertheless, the compound as identified (screened) can be selected from a broad variety of molecules and proteins, and a proteinaceous AHR binding domain, a small molecule, a peptide, environmental substances, probiotics, toxins, aerosols, medicines, nutrients, galenic compositions, plant extracts, volatile compounds, homeopathic substances, incense, pharmaceutical drugs, vaccines, compounds or compound mixtures derived from organisms for example animals, plants, fungi, bacteria, archaea, chemical compounds, and compounds used in food or cosmetic industry, small chemical molecules (less than about 150 Da), peptides, antibodies, and short interfering RNAs or other genetic modifications. Modulators also include proteins that directly or indirectly act on the AHR activation signature, i.e. control or are involved in biological pathways upstream of the AHR activation signature as tested.

Identification and screening can be done using respective methods known in the art, for example using recombinantly produced proteins of the biomarkers, and/or recombinant cell models. For this, the biomarkers can be labeled, e.g. using chemical dyes or fluorescent markers. Also enzymes can be used, for example in the form of fusions with the biomarker to be screened. Preferably, said method is also amenable to automation, and said screening is preferably assessed in an automated and/or high-throughput format.

Because of the known relation of AHR to toxic compounds, another advantageous use is a method for testing the biological safety of a compound, comprising performing a method according to the present invention, and further comprising the step of concluding on the safety of said compound based on said effect as identified.

In another aspect of the present invention, the invention then relates to a diagnostic kit comprising materials for performing a method according to the present invention as herein in one or separate containers, optionally together with auxiliary agents and/or instructions for performing said method according to the present invention. The kit may comprise the panel of biomarkers as identified herein or respective advantageous marker sub-panels as discussed herein. Furthermore, included can be dyes, biomarker-specific antibody, and oligos, e.g. for PCR-assays.

As described herein, in another aspect of the present invention, the invention relates to a panel of biomarkers, about 5, about 10, about 20, about 30 of said AHR biomarkers according to table 1 or at least 10% at least 20%, at least 30%, at least 40%, at least 50%, at least 60%, at least 70%, at least 80% or at least 90% or more or all of said AHR biomarkers according to table 1. Another aspect of the present invention also relates to panels of markers that fulfill certain statistical quality parameters, such as a significance of said AHR activation signature of p < 0.05 at a minimal number of markers in the signature and/or a fold of change of said AHR activation signature of at least about 1.5 at a minimal number of markers in the signature in the case of up-regulation or of at least about 0.67 at a minimal number of markers in the signature in the case of down-regulation.

Further preferred are groups ("panels") of markers that are selected from the above biomarkers (table 1). These panels can be, for example, biomarkers that can be used to identify ones involved in functional groups, such as angiogenesis, drug metabolism, external stress response, hemopoiesis, lipid metabolim, cell motility, and immune modulation, or involved in risk groups, for example, groups of low or high risk for diseases or conditions, e.g. proliferative diseases, cancer, autoimmune disorders and diseases, degeneration, inflammation, infection and intoxication, or an AHR-related function or effect can be selected from at least one of proliferation, toxicity, biological safety screenings, cellular damage or stress, motility, apoptosis, stress conditions, for example, biological, mechanical and environmental stresses and senescence.

Another aspect then relates to the use of at least one biomarker or a panel of biomarkers, in particular 10% or more of the genes according to table 1 for at least one of screening for modulators according to the present invention or for monitoring AHR activation according to the present invention, for testing the biological safety according to the present invention, for screening for a modulator of AHR activation genes or for a diagnosis according to the present invention.

In another preferred aspect of the present invention, the invention then relates to a method of treating and/or preventing an AHR-related disease or condition in a cell in a patient in need of said treatment, comprising performing a method according to the present invention, and providing a suitable treatment to said patient, wherein said treatment is based, at least in part, on the results of the method according to the present invention, such as providing a compound as identified or monitoring a treatment comprising the method(s) as described herein.

Another aspect of the present invention then relates to a method for producing a pharmaceutical preparation, wherein said compound/modulator as identified (screened) is further formulated into a pharmaceutical preparation by admixing said (at least one) compound as identified (screened) with a pharmaceutically acceptable carrier. Pharmaceutical preparations can be preferably present in the form of injectibles, tablets, capsules, syrups, elixirs, ointments, creams, patches, implants, aerosols, sprays and suppositories (rectal, vaginal and urethral). Another aspect of the present invention then relates to a pharmaceutical preparation as prepared according to the invention.

"Treatment" shall mean a reduction and/or amelioration of the symptoms of the disease. An effective treatment achieves, for example, a shrinking of the mass of a tumor and the number of cancer cells. A treatment can also avoid (prevent) and reduce the spread of the cancer, such as, for example, affect metastases and/or the formation thereof. A treatment may be a naive treatment (before any other treatment of a disease had started), or a treatment after the first round of treatment (e.g. after surgery or after a relapse). The treatment can also be a combined treatment, involving, for example, chemotherapy, surgery, and/or radiation treatment. The treatment can also modulate auto-immune response, infection and inflammation.

In the methods of the present invention, in general the biomarkers can be detected and/or determined using any suitable assay. Detection is usually directed at the qualitative information ("marker yes-no"), whereas determining involves analysis of the quantity of a marker (e.g. expression level and/or activity). Detection is also directed at identifying mutations that cause altered functions of individual markers. The choice of the assay(s) depends on the parameter of the marker to be determined and/or the detection process. Thus, the determining and/or detecting can preferably comprise a method selected from subtractive hybridization, microarray analysis, DNA sequencing, qPCR, ELISA, enzymatic activity tests, cell viability assays, for example an MTT assay, phosphoreceptor tyrosine kinase assays, phospho-MAPK arrays and proliferation assays, for example the BrdU assay, proteomics, HPLC and mass spectrometry.

Preferably, said method is also amenable to automation, and said activity and/or expression is preferably assessed in an automated and/or high-throughput format. Usually, this involves the use of chips and respective machinery, such as robots.

Building on previous studies investigating AHR-induced transcription; the inventors compiled gene expression data sets generated through the exposure of cells/tissues to AHR-activating ligands or AHR inhibitors. Additionally, the inventors included target genes with AHR promotor binding sites from the transcription factor binding site database. As experimental validation of all the genes regulated in gene expression datasets is not feasible, the inventors adopted a natural language processing (NLP) approach that extracts genes that share a regulation event with AHR from free texts and abstracts of Pubmed and Pubmed-central.

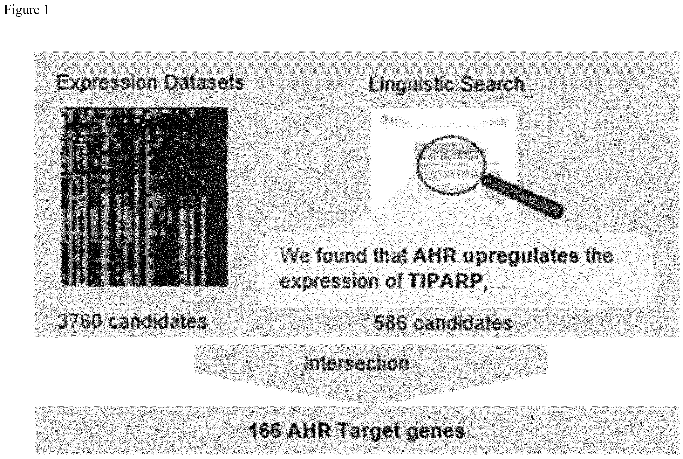

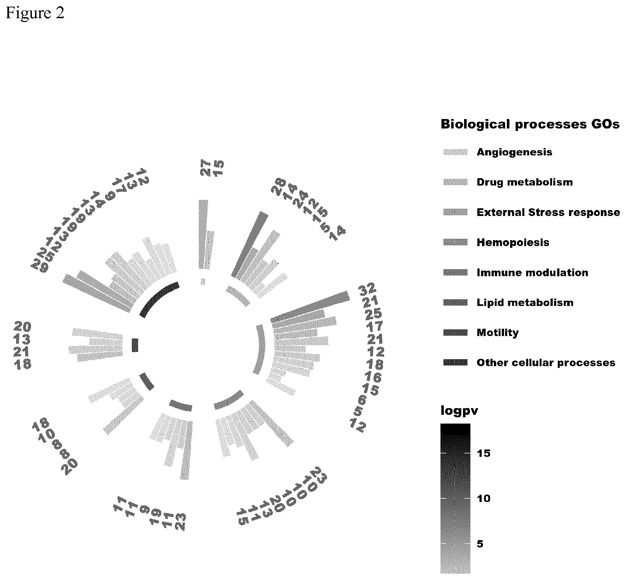

The intersection between the NLP search and the datasets resulted in 166 AHR target genes (Table 1), which is herein referred to as the "AHR signature" (Fig. 1). In support of the inventors' approach, the gene ontology groups enriched in the AHR signature represented the main functions known to be modulated by AHR including drug metabolism, cell motility and immune modulation (Fig. 2).

**Table 1 - AHR target genes and AHR signature; genes are indicated with their HGNC approved name and Entrez database ID for human copies**

| **Gene** | **Entrez ID (homo sapiens)** |
|---|---|
| actin alpha 2, smooth muscle (ACTA2) | 59 |
| adhesion molecule with Ig like domain 2 (AMIGO2) | 347902 |
| adrenomedullin (ADM) | 133 |
| aldehyde dehydrogenase 3 family member A1 (ALDH3A1) | 218 |
| amphiregulin (AREG) | 374 |
| aquaporin 3 (Gill blood group) (AQP3) | 360 |
| arginase 2 (ARG2) | 384 |
| aryl hydrocarbon receptor (AHR) | 196 |
| aryl-hydrocarbon receptor repressor (AHRR) | 57491 |
| ATP binding cassette subfamily C member 4 (ABCC4) | 10257 |
| ATP binding cassette subfamily G member 2 (Junior blood group) (ABCG2) | 9429 |
| ATP synthase inhibitory factor subunit 1 (ATP5IF1) | 93974 |
| ATP synthase membrane subunit e (ATP5ME) | 521 |
| ATPase H+ transporting accessory protein 2 (ATP6AP2) | 10159 |
| ATPase H+/K+ transporting non-gastric alpha2 subunit (ATP12A) | 479 |
| B cell linker (BLNK) | 29760 |
| BAF chromatin remodeling complex subunit BCL11B (BCL11B) | 64919 |
| BCL2 apoptosis regulator (BCL2) | 596 |
| BCL6 transcription repressor (BCL6) | 604 |
| BRCA1 DNA repair associated (BRCA1) | 672 |
| C-C motif chemokine ligand 5 (CCL5) | 6352 |
| C-X-C motif chemokine ligand 2 (CXCL2) | 2920 |
| caspase recruitment domain family member 11 (CARD11) | 84433 |
| caveolin 1 (CAV1) | 857 |
| CD3e molecule (CD3E) | 916 |
| CD36 molecule (CD36) | 948 |
| CD8a molecule (CD8A) | 925 |
| coagulation factor III, tissue factor (F3) | 2152 |
| corticotropin releasing hormone (CRH) | 1392 |
| cyclin D1 (CCND1) | 595 |
| cyclin dependent kinase 4 (CDK4) | 1019 |
| cyclin dependent kinase inhibitor 1A (CDKN1A) | 1026 |
| cystic fibrosis transmembrane conductance regulator (CFTR) | 1080 |
| cytochrome b-245 beta chain (CYBB) | 1536 |
| cytochrome P450 family 1 subfamily A member 1 (CYP1A1) | 1543 |
| cytochrome P450 family 1 subfamily A member 2 (CYP1A2) | 1544 |
| cytochrome P450 family 1 subfamily B member 1 (CYP1B1) | 1545 |
| cytochrome P450 family 19 subfamily A member 1 (CYP19A1) | 1588 |
| cytochrome P450 family 2 subfamily B member 6 (CYP2B6) | 1555 |
| cytochrome P450 family 2 subfamily E member 1 (CYP2E1) | 1571 |
| cytochrome P450 family 3 subfamily A member 4 (CYP3A4) | 1576 |
| dickkopf WNT signaling pathway inhibitor 3 (DKK3) | 27122 |
| distal-less homeobox 3 (DLX3) | 1747 |
| DNA polymerase kappa (POLK) | 51426 |
| dual oxidase 2 (DUOX2) | 50506 |
| early growth response 1 (EGR1) | 1958 |
| EBF transcription factor 1 (EBF1) | 1879 |
| endothelin 1 (EDN1) | 1906 |
| epidermal growth factor receptor (EGFR) | 1956 |
| epiregulin (EREG) | 2069 |
| epithelial mitogen (EPGN) | 255324 |
| estrogen receptor 1 (ESR1) | 2099 |
| F-box protein 32 (FBXO32) | 114907 |
| Fas cell surface death receptor (FAS) | 355 |
| FAT atypical cadherin 1 (FAT1) | 2195 |
| fibroblast growth factor receptor 2 (FGFR2) | 2263 |
| FIG4 phosphoinositide 5-phosphatase (FIG4) | 9896 |
| filaggrin (FLG) | 2312 |
| forkhead box A1 (FOXA1) | 3169 |
| forkhead box Q1 (FOXQ1) | 94234 |
| formyl peptide receptor 2 (FPR2) | 2358 |
| Fos proto-oncogene, AP-1 transcription factor subunit (FOS) | 2353 |
| G protein subunit alpha 13 (GNA13) | 10672 |
| GATA binding protein 3 (GATA3) | 2625 |
| glutamine amidotransferase like class 1 domain containing 3A (GATD3A) | 8209 |
| glutathione S-transferase alpha 2 (GSTA2) | 2939 |
| glutathione S-transferase mu 1 (GSTM1) | 2944 |
| growth factor independent 1 transcriptional repressor (GFI1) | 2672 |
| growth hormone receptor (GHR) | 2690 |
| heat shock protein family B (small) member 2 (HSPB2) | 3316 |
| heme oxygenase 1 (HMOX1) | 3162 |
| hes family bHLH transcription factor 1(HES1) | 3280 |
| hydroxysteroid 17-beta dehydrogenase 4 (HSD17B4) | 3295 |
| hypoxia inducible factor 1 subunit alpha (HIF1A) | 3091 |
| IKAROS family zinc finger 3 (IKZF3) | 22806 |
| inhibitor of DNA binding 1, HLH protein (ID1) | 3397 |
| inhibitor of DNA binding 2 (ID2) | 3398 |
| insulin induced gene 1 (INSIG1) | 3638 |
| insulin like growth factor 2 (IGF2) | 3481 |
| insulin like growth factor binding protein 1(IGFBP1) | 3484 |
| interferon gamma (IFNG) | 3458 |
| interferon regulatory factor 8 (IRF8) | 3394 |
| interleukin 1 beta (IL1B) | 3553 |
| interleukin 1 receptor type 2 (IL1R2) | 7850 |
| interleukin 2 (IL2) | 3558 |
| interleukin 6 (IL6) | 3569 |
| jagged canonical Notch ligand 1 (JAG1) | 182 |
| junction plakoglobin (JUP) | 3728 |
| KIAA1549 (KIAA1549) | 57670 |
| KIT proto-oncogene, receptor tyrosine kinase (KIT) | 3815 |
| kynurenine 3-monooxygenase (KMO) | 8564 |
| latent transforming growth factor beta binding protein 1 (LTBP1) | 4052 |
| leptin receptor (LEPR) | 3953 |
| LIF receptor alpha (LIFR) | 3977 |
| lipoprotein lipase (LPL) | 4023 |
| luteinizing hormone/choriogonadotropin receptor (LHCGR) | 3973 |
| LYN proto-oncogene, Src family tyrosine kinase (LYN) | 4067 |
| lysine demethylase 1A (KDM1A) | 23028 |
| major histocompatibility complex, class II, DR beta 4 (HLA-DRB4) | 3126 |
| matrix metallopeptidase 1 (MMP1) | 4312 |
| midline 1 (MIDI) | 4281 |
| musashi RNA binding protein 2 (MSI2) | 124540 |
| MYC proto-oncogene, bHLH transcription factor (MYC) | 4609 |
| N-myc downstream regulated 1 (NDRG1) | 10397 |
| NAD(P) dependent steroid dehydrogenase-like (NSDHL) | 50814 |
| NAD(P)H quinone dehydrogenase 1 (NQO1) | 1728 |
| Nanog homeobox (NANOG) | 79923 |
| neural precursor cell expressed, developmentally down-regulated 9 (NEDD9) | 4739 |
| neuronal pentraxin 1 (NPTX1) | 4884 |
| nitric oxide synthase 1 (NOS1) | 4842 |
| nitric oxide synthase 3 (NOS3) | 4846 |
| nuclear factor, erythroid 2 like 2 (NFE2L2) | 4780 |
| nuclear receptor coactivator 2 (NCOA2) | 10499 |
| nuclear receptor corepressor 2 (NCOR2) | 9612 |
| nuclear receptor interacting protein 1 (NRIP1) | 8204 |
| nuclear receptor subfamily 1 group H member 3 (NR1H3) | 10062 |
| nuclear receptor subfamily 1 group H member 4 (NR1H4) | 9971 |
| nuclear receptor subfamily 3 group C member 1 (NR3C1) | 2908 |
| ovo like transcriptional repressor 1 (OVOL1) | 5017 |
| paired box 5 (PAX5) | 5079 |
| patatin like phospholipase domain containing 7 (PNPLA7) | 375775 |
| PDS5 cohesin associated factor B (PDS5B) | 23047 |
| period circadian regulator 1 (PERI) | 5187 |
| phosphodiesterase 2A (PDE2A) | 5138 |
| phosphoenolpyruvate carboxykinase 1 (PCK1) | 5105 |
| phosphoenolpyruvate carboxykinase 2, mitochondrial (PCK2) | 5106 |
| phosphoglycerate dehydrogenase (PHGDH) | 26227 |
| phospholipase A2 group IVA (PLA2G4A) | 5321 |
| piwi like RNA-mediated gene silencing 1 (PIWIL1) | 9271 |
| piwi like RNA-mediated gene silencing 2 (PIWIL2) | 55124 |
| PPARG coactivator 1 alpha (PPARGC1A) | 10891 |
| PR/SET domain 1 (PRDM1) | 639 |
| prostaglandin-endoperoxide synthase 2 (PTGS2) | 5743 |
| R-spondin 3 (RSPO3) | 84870 |
| REL proto-oncogene, NF-kB subunit (REL) | 5966 |
| replication factor C subunit 3 (RFC3) | 5983 |
| retinoic acid receptor alpha (RARA) | 5914 |
| scavenger receptor class B member 1 (SCARB1) | 949 |
| scinderin (SCIN) | 85477 |
| serpin family B member 2 (SERPINB2) | 5055 |
| serpin family E member 1 (SERPINE1) | 5054 |
| sestrin 2 (SESN2) | 83667 |
| SH3 domain containing kinase binding protein 1 (SH3KBP1) | 30011 |
| SMAD family member 3 (SMAD3) | 4088 |
| SMAD family member 7 (SMAD7) | 4092 |
| small proline rich protein 2D (SPRR2D) | 6703 |
| solute carrier family 10 member 1 (SLC10A1) | 6554 |
| solute carrier family 3 member 2 (SLC3A2) | 6520 |
| solute carrier family 7 member 5 (SLC7A5) | 8140 |
| sortilin related receptor 1 (SORL1) | 6653 |
| SOS Ras/Rac guanine nucleotide exchange factor 1 (SOS1) | 6654 |
| stanniocalcin 2 (STC2) | 8614 |
| suppressor of cytokine signaling 2 (SOCS2) | 8835 |
| TCDD inducible poly(ADP-ribose) polymerase (TIPARP) | 25976 |
| thioredoxin reductase 1 (TXNRD1) | 7296 |
| thrombospondin 1 (THBS1) | 7057 |
| tight junction protein 1 (TJP1) | 7082 |
| TNF superfamily member 9 (TNFSF9) | 8744 |
| transforming growth factor beta induced (TGFBI) | 7045 |
| transglutaminase 1 (TGM1) | 7051 |
| trefoil factor 1 (TFF1) | 7031 |
| tyrosine hydroxylase (TH) | 7054 |
| UDP glucuronosyltransferase family 1 member A6 (UGT1A6) | 54578 |
| vav guanine nucleotide exchange factor 3 (VAV3) | 10451 |
| xanthine dehydrogenase (XDH) | 7498 |
| Zic family member 3 (ZIC3) | 7547 |

The AHR signature was further validated using *roast* gene set enrichment in distinct datasets of cells treated with TCDD (Fig. 3), the AHR inhibitors SRI (Fig. 4a-c), or CH223191 (Fig. 4d), as well as the endogenous AHR agonists 6-formylindolo(3,2b)carbazole (FICZ) and kynurenine, kynurenic acid and indole-3-carboxaldehyde (Fig. 5 and 6).

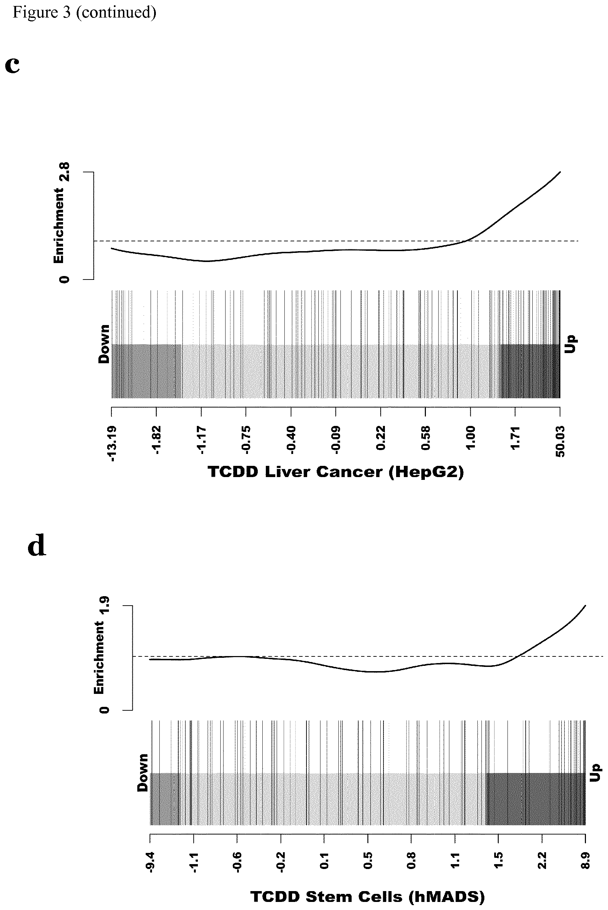

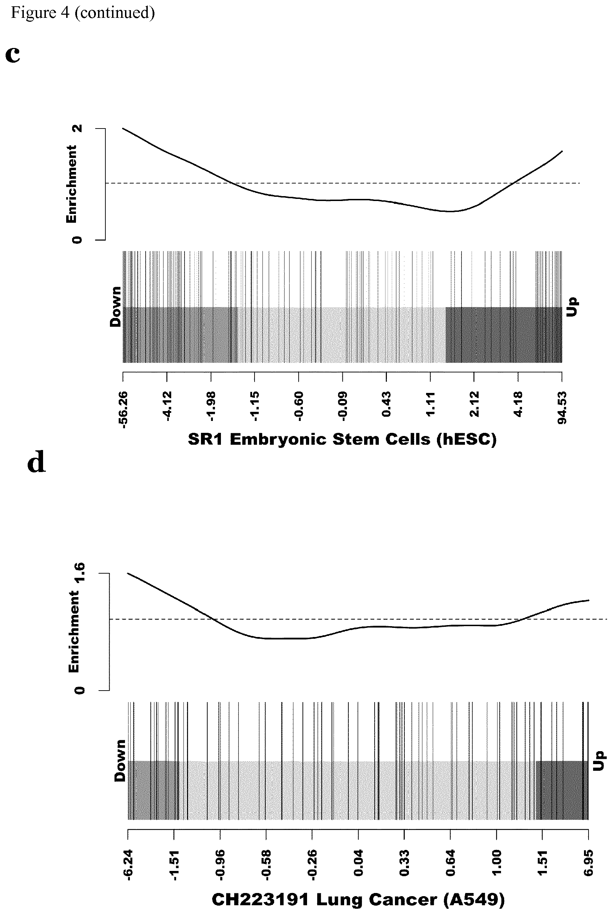

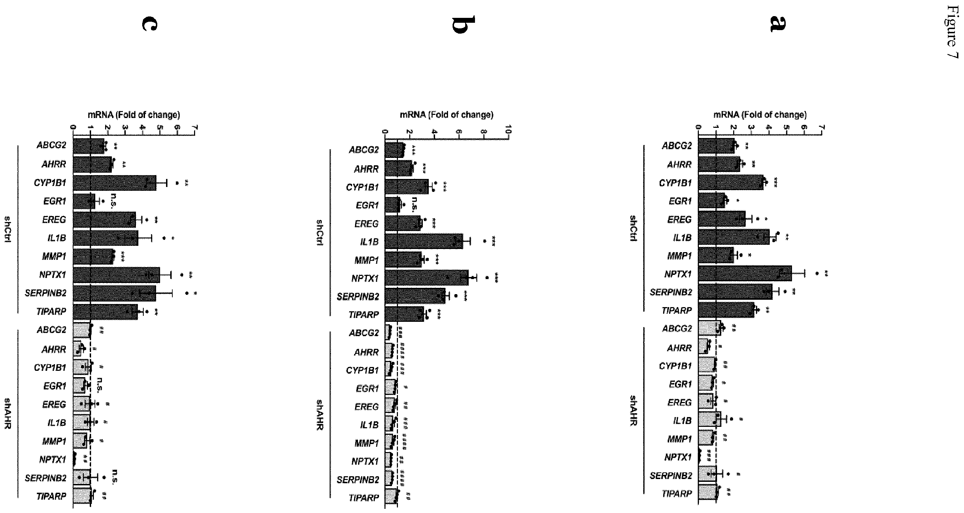

In addition, the inventors performed qRT-PCRs of selected signature genes in conditions of AHR activation with TCDD, FICZ or Kyn as well as combined ligand activation and AHR knockdown (Fig 7). Owing to the cell/tissue and ligand specificity of AHR target gene expression, the inventors confirmed that the AHR signature is able to detect modulation of AHR activity also in cell types (Fig. 3d; Fig. 4a-c; Fig.5; Fig 6) and in response to ligands (Fig. 4d, Fig. 5, Fig. 6 and Fig. 7b,c) that were not employed to generate the AHR signature.

The AHR signature as established is also capable of detecting AHR activation across different cell/tissue types and in response to diverse ligands. Using the AHR signature, it is possible to determine AHR activation sub-groups by unsupervised clustering methods, which can be utilized for classification of samples. This is important for example, in terms of selecting patients for treatment with AHR activation modulating interventions, and monitoring of therapy response.

The invention shall now be further described in the following examples with reference to the accompanying Figures, nevertheless, without being limited thereto. For the purposes of the present invention, all references as cited herein are incorporated by reference in their entireties.
Figure 1 shows a diagram of the workflow for generating the AHR signature. The graphical representation describes the generation of the AHR signature by integrating results of natural language processing of free full texts and abstracts of PubMed and PubMed-Central, and mined gene expression datasets.
Figure 2 shows a circular bar graph representing eight biological processes gene ontology groups that are enriched in the AHR signature genes. The inner most circle represents the color code of each ontology groups. Each bar represents a significantly enriched ontology term. The bars are ordered in a descending order of highest significance in a clockwise fashion. The colors of each bar correspond to the significance of enrichment (logpv = -log10 p-value of enrichment). The length of each bar and the numbers in the outer circle represent the number of genes from the AHR signature sharing the same ontology term.

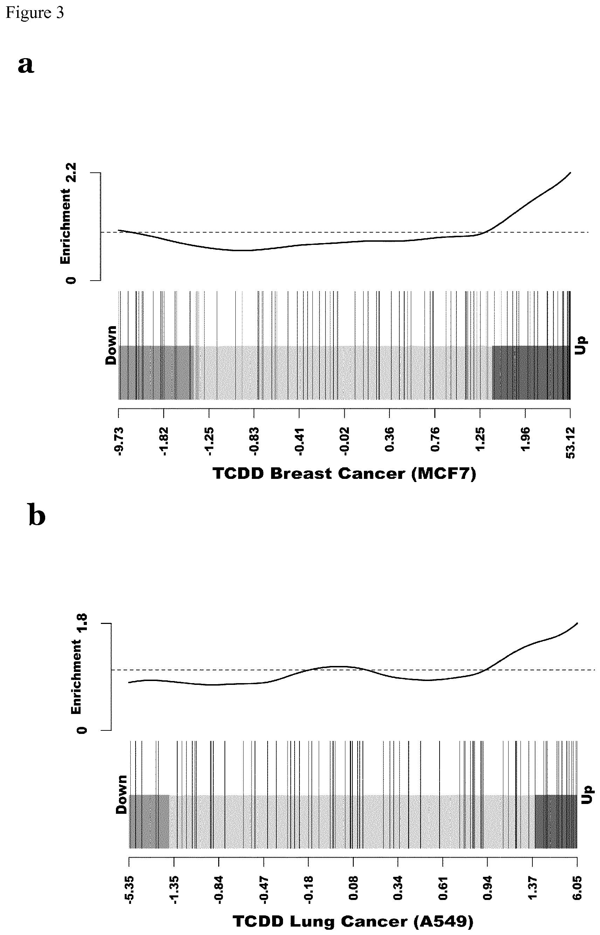

Figure 3 shows barcode plots showing the direction of regulation of the AHR signature after performing differential gene regulation of: a) MCF7 cells exposed to 100 nM TCDD for 24 hours compared to DMSO (GSE98515), b) A549 cells exposed to 10 nM TCDD for six hours (GSE109576), c) HepG2 cells exposed to 10 nM TCDD for 24 hours (GSE28878), d) Human Multipotent Adipose-Derived Stem cells (hMADS) exposed to 25 nM TCDD for 24 hours (GSE32026). The x-axis represents the moderated t-statistic values for all genes in the comparison. The darker grey scales represent the lower and upper quartiles of all the genes. The vertical barcode lines represent the distribution of the AHR signature genes. The worm line representation above the barcode shows the direction of regulation of the AHR signature.

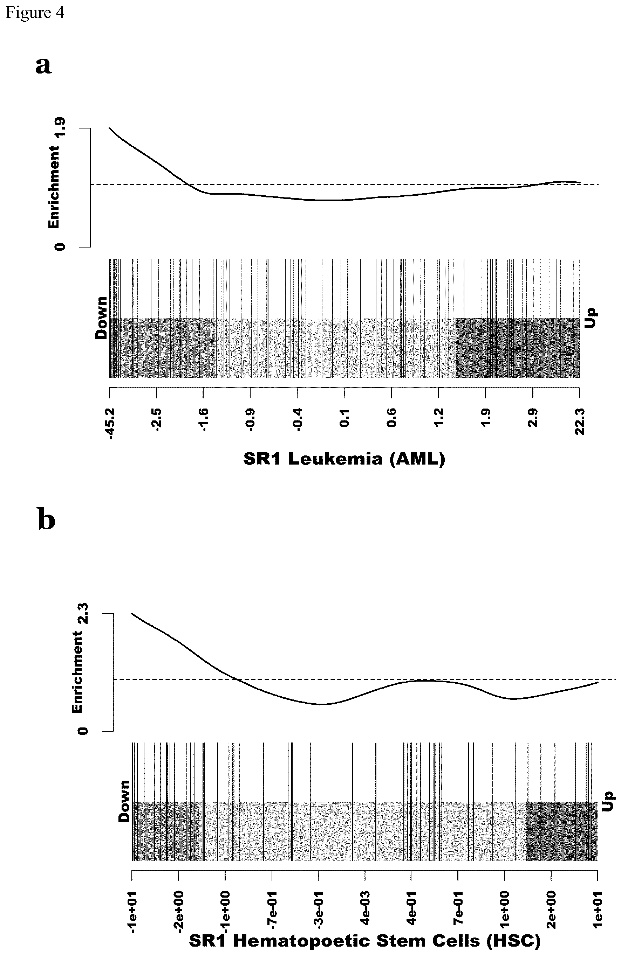

Figure 4 shows barcode plots showing the direction of regulation of the AHR signature after performing differential gene regulation of: a) primary AML cells exposed to 500 nM SRI for 16 hours (GSE48843), b) CD34 positive hematopoietic stem cells (HSC) treated with 1 uM SRI for 7 days (GSE67093), c) hESC cells treated with SRI for 24 hours (GSE52158), d) A549 cells exposed to 10 uM CH223191 for six hours (GSE109576).

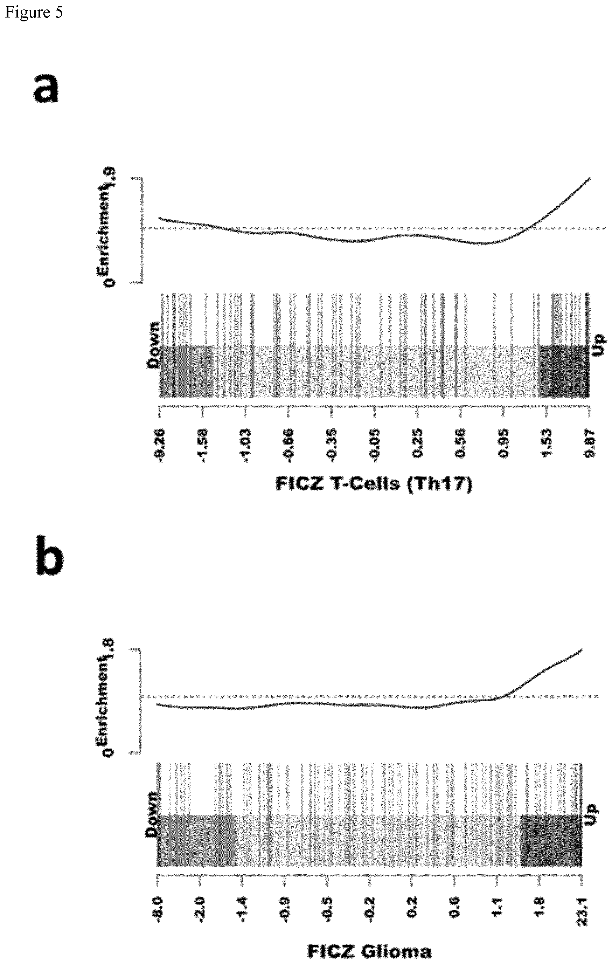

Figure 5 shows barcode plots showing the direction of regulation of the AHR signature after performing differential gene regulation of: a) Th17 cells exposed to 200 nM FICZ for 16 hours (GSE102045), b) U87 cells exposed to 100 nM FICZ for 24 hours.

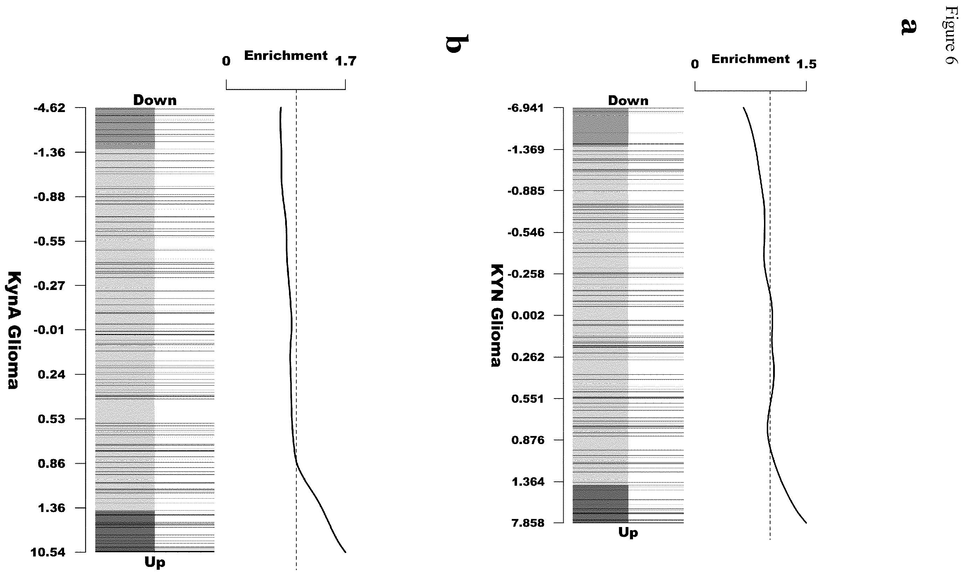

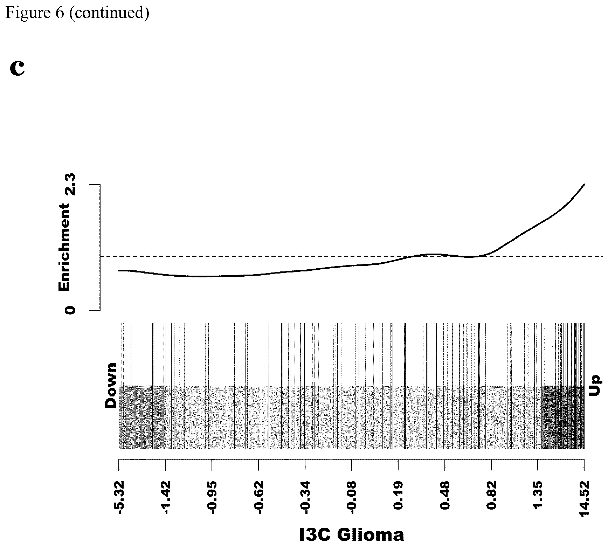

Figure 6 shows barcode plots showing the direction of regulation of the AHR signature after performing differential gene regulation of: a) U87 cells exposed to 100 uM Kyn for 8 hours (GSE25272), b) U87 cells exposed to 50 uM KynA for 24 hours, c) U87 cells exposed to 50 uM I3CA for 24 hours.
Figure 7 shows a) mRNA expression of selected AHR target genes in U-87MG-shCtrl (shCtrl) and U-87MG-shAHR (shAHR) treated with 10 nM TCDD or vehicle for 24 h (n = 3). b) mRNA expression of selected AHR target genes in U-87MG-shCtrl and U-87MG-shAHR treated with 100 nM FICZ or vehicle for 24 h (n = 4). c) mRNA expression of selected AHR target genes in U-87 MG-shCtrl and U-87MG-shAHR treated with 50 µM Kyn or vehicle for 24 h (n = 3). n values represent the number of independent experiments. Data represented as mean ± S.E.M and were analyzed by two-tailed paired student's t-test (e, j). *P < 0.05, ** P < 0.01, *** P < 0.001, **** P < 0.0001. n.s., not significant. * vehicle compared to treatment; # treatment in shC compared to shAHR

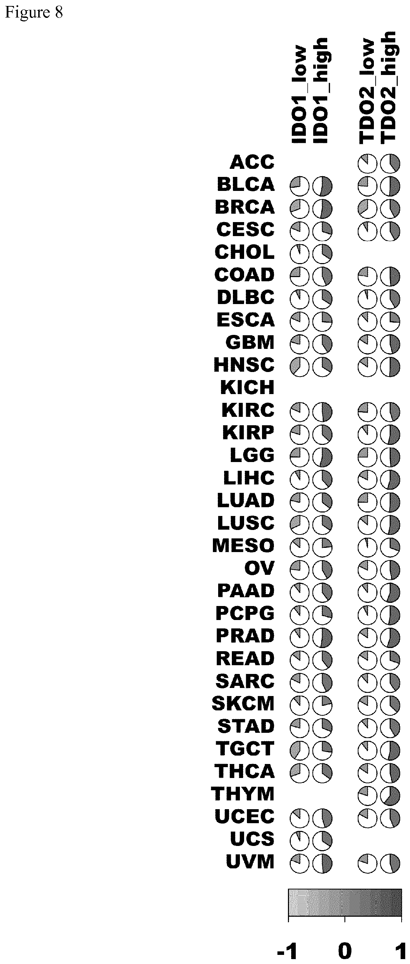

Figure 8 shows pie chart representations showing the results of gene set enrichment using *roast* on patients of 32 primary TCGA tumors after median separation of the patients into groups of high and low expression of IDO1 or TDO2. The missing piecharts designate that there was no significant AHR modulation detected in the high-low group comparisons. The darker color shades in the pie charts show the percentage of up-regulated AHR signature genes in the high-low comparisons, and subsequently, the lighter color shades show the percentage of down-regulated AHR signature genes in the high-low comparisons. The sum of the shaded pie chart segments denotes the percentage of AHR signature genes that were differentially regulated.

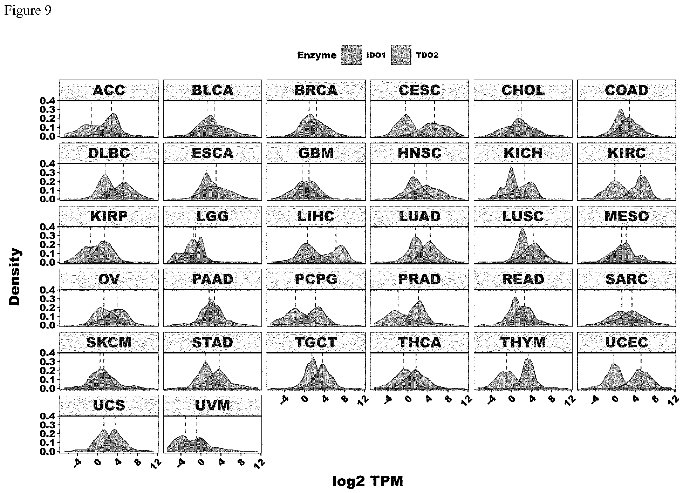

Figure 9 shows density plots showing multi-modal distributions of the log2 transcripts per million (log2 TPM) expression levels of IDO1 (light grey) and TDO2 (dark grey) in 32 primary TCGA tumors. The vertical dotted lines show the median value for IDO1 (light grey) or TDO2 (dark grey).

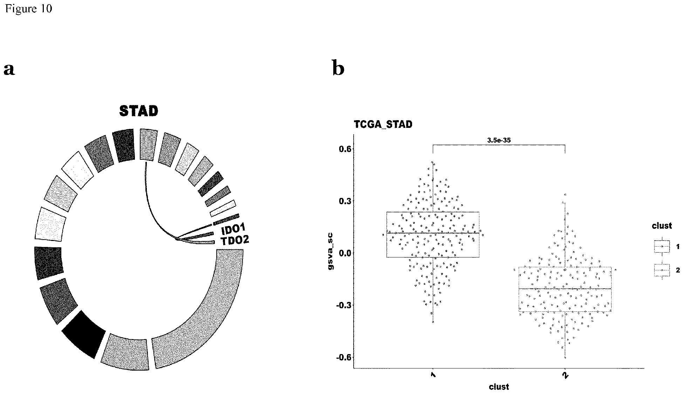

Figure 10: shows a) Circos showing the connections of IDO1 and TDO2 if co-expressed in WGCNA modules, positively associated with AHR activation in Stomach adenocarcinoma (STAD). The circular segments correspond to the WGCNA module, and the connections have the same color as the corresponding module. The size of the module is proportionate to the number of genes. b) Box plot representation of the AHR activation score in STAD subtypes. Group comparison was performed by a Wilcox-sum rank test.

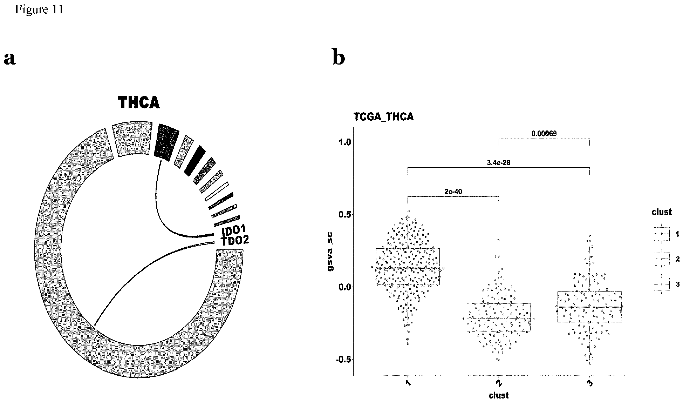

Figure 11 shows: a) Circos showing the connections of IDO1 and TDO2 if co-expressed in WGCNA modules, positively associated with AHR activation in Thyroid carcinoma (THCA). The circular segments correspond to the WGCNA module, and the connections have the same color as the corresponding module. The size of the module is proportionate to the number of genes. b) Box plot representation of the AHR activation score in THCA subtypes. Group comparisons were performed by a Wilcox-sum rank test.

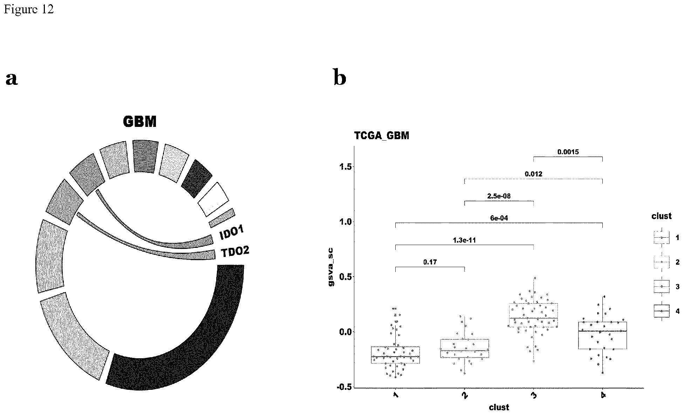

Figure 12 shows: a) Circos showing the connections of IDO1 and TDO2 if co-expressed in WGCNA modules, positively associated with AHR activation in Glioblastoma multiforme (GBM). The circular segments correspond to the WGCNA module, and the connections have the same color as the corresponding module. The size of the module is proportionate to the number of genes. b) Box plot representation of the AHR activation score in GBM subtypes. Group comparisons were performed by a Wilcox-sum rank test.

SEQ ID Nos 1 to 3 show shAHR sequences for knockdown experiments.

SEQ ID Nos 4 to 25 show oligonucleotide sequences for rtPCR experiments.

### Examples

### Material and Methods

### Generating the AHR gene transcriptional/activation signature

First, existing datasets for different AHR activation or inhibition conditions were identified in the GEO database (1). The search was performed using an in-house tool using several keywords. The list of datasets was manually curated and a cutoff for differentially expressed genes was set at log2 fold change of 0.3 (and an adjusted p-value threshold of 0.05). In addition, AHR targets were retrieved from the Transcription Factor Target Gene Database (2) and merged with the gene list curated from the GEO search.

A semantic analysis was carried out to correctly identify the appearance of gene names including AHR in given freely available free texts with GeNo (3) and gene interactions, called events, using BioSem (4). The output of BioSem was then stored in an ElasticSearch index (https://www.elastic.co/). From this index, event items referencing AHR as an interaction member with a regulation event were selected. Results were manually curated to obtain the final list of literature mentioning AHR associated interaction events. Human orthologues were used to replace mouse genes in the NLP search results. The gene annotations of both text mining and dataset searches results were harmonized by cross referencing with the accepted HGNC symbols (www.hgnc.com) as per the hg38 reference. Genes overlapping between the two lists were used to constitute the core AHR activation signature consisting of 166 genes (Table 1).

### Annotation of the AHR gene transcriptional/activation signature

Gene ontology analysis of the core AHR activation signature was performed using the clusterProfiler package (5), applying the method described by Boyle et al. (2004) (6). Bonferroni correction was used to control for multiple testing and a p-value cutoff of 0.01 was used for selecting enriched ontology terms. The semantic similarity algorithm GOsemsim (7) was used for grouping of ontology terms followed by filtering of higher/general levels ontology term. The remaining ontology terms were categorized into eight groups descriptive of AHR activation mediated biological processes.

### Microarray and RNA-seq data analysis

Array datasets - The Affymetrix microarray chips "human gene 2.0 ST" were analyzed using the oligo package and annotated using NetAffx (8). Other Affymetrix chips were analyzed using the Affy and Affycoretools packages. Raw CEL files were imported from disk or downloaded from Gene Expression Omnibus (GEO) using GEOquery (9), followed by RMA normalized and summarization. Illumina and Agilent array datasets were analyzed using lumi (10-11) and limma (12).

RNA-seq datasets - Raw counts and metadata were downloaded from GEO using GEOquery and saved as a DGElist (13). The harmonized HT-Seq counts of TCGA datasets were downloaded using TCGAbiolinks (14) from GDC (http://gdc-api.nci.nih.gov), and only patients with the identifier "primary solid tumor" were retained, with the exception of melanoma that was split into datasets for primary and advanced melanoma cohorts. Genes with less than 10 counts were filtered followed by TMM normalization (15) and variance modelling using voom (15).

### Differential gene expression and gene set testing

The eBayes adjusted moderated t-statistic was applied for differential gene expression using *limma* (12) and limma-trend (17) or the limma RNA-seq pipeline (12). Batch effects, when present, were accounted for in the linear regression. Gene set testing of AHR activation was performed using *roast* (18).

### Association of AHR activation with patient groups of median separated enzyme expression

Assessing the association of AHR activation with Trp degrading enzymes, TCGA patients were divided by the median into groups of high or low expression of IDO1 or TDO2, and differential gene expression and gene set testing was conducted as described above.

### AHR activation score

Using the AHR signature, the single sample gene set enrichment scores was estimated using the GSVA package (19), we refer to as the AHR activation score.

### Gene correlation networks associated with AHR activation

The normalized DGEList of publicly available GEO data was used for weighted gene co-expression network analysis (WGCNA) (20). Soft thresholds were estimated for signed hybrid networks in single block settings. Adjacency and topology overlapping matrices were calculated using bi-correlation matrices and Eigen genes representing the first principle components of each module were returned. Selecting WGCNA modules associated with AHR activation was conducted by performing a global test (21-23) using the AHR activation score as the response and the WGCNA modules as model predictors. Additionally, using Pearson correlation, as implemented in the Hmisc package (https://cran.r-project.org/web/packages/Hmisc/index.html), AHR activation scores were correlated with WGCNA modules. Modules that overlapped the global test and Pearson correlation results, with a p-value of 0.05 or less in both tests, were selected as the AHR associated modules, regardless of the direction of association, i.e. both positively and negatively associated modules were retained if overlapping and satisfying the p-value cutoff.

### Defining AHR activation sub-groups

K-means consensus clustering (24-25), using AHR associated modules, was performed to define patient subgroups with AHR activation. The number of clusters for each tumor type was assessed using consensus heatmaps, cumulative distribution function plots, elbow plots and samples' cluster identities. The values of K explored were 2-20, with k=2-4 providing the most stable clusters. The group separation was further examined using principle component analysis.

### Cell culture

U-87MG were obtained from ATCC. U-87MG were cultured in phenol red-free, high glucose DMEM medium (Gibco, 31053028) supplemented with 10% FBS (Gibco, 10270106), 2 mM L-glutamine, 1 mM sodium pyruvate, 100 U/mL penicillin and 100 µg/mL streptomycin (referred to as complete DMEM). Cell lines were cultured at 37 °C and 5 % CO₂. Cell lines were authenticated and certified to be free of mycoplasma contamination.

### Cell culture Treatment conditions

For treatment of adherent cells with AHR ligands, 4 x 10⁵ cells per well were seeded in six well plates and incubated for 24 h prior to treatment. Non-adherent cells were seeded at 5 x 10⁵ cells/mL in 24 well plates and treated immediately. For verification of the generated AHR signature, cells were treated with the established AHR agonists TCDD (10 nM, American Radiolabeled Chemicals Inc.,), FICZ (100 nM, Cayman Chemicals, 19529), Kyn (50 µM, Sigma Aldrich), KynA (50 uM, Sigma-Aldrich, K3375) and indole-3-carboxaldehyde (6.25 µM to 100 µM, Sigma-Aldrich, 129445) for 24 h.

**Stable knockdown of U-87MG cells**Stable knockdown of AHR in U-87MG cells was achieved using shERWOOD UltramiR Lentiviral shRNA targeting AHR (transOMIC Technologies, TLHSU1400-196-GVO-TRI). Glioma cells were infected with viral supernatants containing either shAHR or shControl (shC) sequences to generate stable cell lines. Both shAHR sequences displayed similar knockdown efficiency and stable cell lines with shAHR#1 were used for experiments.

shERWOOD UltramiR shRNA sequences are:

shAHR#1 (ULTRA-3234821): 5'-
TGCTGTTGACAGTGAGCGCAGGAAGAATTGTTT
TAGGATATAGTGAAGCCACAGATGTATATCCTAAAACAATTCTTCCTTTGCC
TACTGCCTCGGA-3' (SEQ ID NO: 1);

shAHR#2 (ULTRA-3234823): 5'-TGCTGTTGACAGTGAGCGCCC CAC
AAGATGTTATTAATAATAGTGAAGCCACAGATGTATTATTAATAACATCTTG
TG GGATGCCTACTGCCTCGGA-3' (SEQ ID NO:2);

shC (ULTRA-NT#4): 5’- TGCTGTTGACAGTGA GCG
AAGGCAGAAGTATGCAAAGCATTAGTGAAGCCACAGATGTAATGCTTTGCA
TACTTCTGCCTGTGCCTACTGCCTCGGA-3’ (SEQ ID NO: 3).

### RNA isolation and real time PCR

Total RNA was harvested from cultured cells using the RNeasy Mini Kit (Qiagen) followed by cDNA synthesis using the High Capacity cDNA reverse transcriptase kit (Applied Biosystems). StepOne Plus real-time PCR system (Applied Biosystems) was used to perform real time PCR of cDNA samples using SYBR Select Master mix (Thermo Scientific). Data was processed and analysed using the StepOne Software v 2.3. Relative quantification of target genes was done against *RNA18S* as reference gene using the 2^{ΔΔCt} method. Human primer sequences are,
*18S RNA-Fwd 5*'-GATGGGCGGCGGAAAATAG-3' (SEQ ID NO: 4),
*18S RNA-Rev 5*'-GCGTGGATTCTGCATAATGGT-3' (SEQ ID NO: 5),
*IL1B-Fwd 5*'-CTCGCCAGTGAAATGATGGCT-3' (SEQ ID NO: 6),
*IL1B-Rev 5*'-GTCGGAGATTCGTAGCTGGAT-3' (SEQ ID NO: 7),
*CYP1B1-Fwd 5*'-GACGCCTTTATCCTCTCTGCG-3' (SEQ ID NO: 8),
*CYP1B1-Rev 5*'-ACGACCTGATCCAATTCTGCCCA-3' (SEQ ID NO: 9),
*EREG-Fwd 5*'-CTGCCTGGGTTTCCATCTTCT-3' (SEQ ID NO: 10),
*EREG-Rev 5*'-GCCATTCATGTCAGAGCTACACT-3' (SEQ ID NO: 11),
*NPTX1-Fwd 5*'-CATCAATGACAAGGTGGCCAAG-3' (SEQ ID NO: 12),
*NPTX1-Rev 5*'-GGGCTTGATGGGGTGATAGG-3' (SEQ ID NO: 13),
*SERPINEB2-Fwd 5*'-ACCCCCATGACTCCAGAGAA-3' (SEQ ID NO: 14),
*SERPINEB2-Rev 5*'-CTTGTGCCTGCAAAATCGCAT-3' (SEQ ID NO: 15),
*TIPARP-Fwd 5*'-CACCCTCTAGCAATGTCAACTC-3' (SEQ ID NO: 16),
*TIPARP-Rev 5*'-CAGACTCGGGATACTCTCTCC-3' (SEQ ID NO: 17),
*MMP1-Fwd 5*'-GCTAACCTTTGATGCTATAACTACGA-3' (SEQ ID NO: 18),
*MMP1-Rev 5*'-TTTGTGCGCATGTAGAATCTG-3' (SEQ ID NO: 19),
*AHRR-Fwd 5*'-CCCTCCTCAGGTGGTGTTTG-3' (SEQ ID NO: 20),
*AHRR-Rev 5*'-CGACAAATGAAGCAGCGTGT-3' (SEQ ID NO: 21),
*ABCG2-Fwd 5*'-TTCCACGATATGGATTTACGG-3' (SEQ ID NO: 22),
*ABCG2-Rev 5*'-GTTTCCTGTTGCATTGAGTCC-3' (SEQ ID NO: 23),
*EGR1-Fwd 5*'-CTGACCGCAGAGTCTTTTCCT-3' (SEQ ID NO: 24), and
*EGR1-Rev 5*'-GAGTGGTTTGGCTGGGGTAA-3' (SEQ ID NO: 25).

### Software and statistics

Graphical and statistical analysis of gene (real time-PCR) was done using GraphPad Prism software versions 6.0 and 8.0. Unless otherwise indicated, data represents the mean ± S.E.M of at least 3 independent experiments. In cases where data was expressed as absolute fold of change, these values were Log10 transformed and the resulting values were used for statistical analysis. Depending on the data, the following statistical analyses were applied: two-tailed student's t-test (paired or unpaired) and repeated measures ANOVA with Dunnett's multiple comparisons test. Significant differences were reported as *p < 0.05, ** p < 0.01, *** p < 0.001, **** p < 0.0001. NS indicates no significant difference. For bioinformatics analysis, unless stated otherwise, all pairwise comparisons were performed using Kruskal-Wallis and Wilcoxon rank sum test, and all reported p-values were adjusted using the Benjamini-Hochberg procedure. All analyses were run in R, version 3.4.4, (https://cran.r-project.org/) and Bioconductor version 3.6 (https://bioconductor.org/). All graphical representations were generated using ggplot2, ggpubr, corrplot, gplots, gridExtra, ComplexHeatmap (26), circlize (27), factoextra, and RcolorBrewer.

### References as cited

**1.** Edgar R, Domrachev M, Lash AE. Gene Expression Omnibus: NCBI gene expression and hybridization array data repository Nucleic Acids Res. 2002 Jan 1;30(1):207-10.
**2.** Plaisier CL, et al. Causal Mechanistic Regulatory Network for Glioblastoma Deciphered Using Systems Genetics Network Analysis. Cell Syst. 2016 Aug;3(2):172-86.
**3.** Wermter, J., Tomanek, K. & Hahn, U. High-performance gene name normalization with GeNo. Bioinformatics 25, 815-821 (2009).
**4.** Bui, Q.-C. & Sloot, P. M. A. A robust approach to extract biomedical events from literature. Bioinformatics 28, 2654-2661 (2012).
**5.** Yu, Guangchuang, et al. 2012. "ClusterProfiler: An R Package for Comparing Biological Themes Among Gene Clusters." OMICS: A Journal of Integrative Biology 16 (5):284-87. https://doi.org/10.1089/omi.2011.0118.
**6.** Boyle, Elizabeth I, et al. 2004. "GO: TermFinder-open Source Software for Accessing Gene Ontology Information and Finding Significantly Enriched Gene Ontology Terms Associated with a List of Genes." Bioinformatics (Oxford, England) 20 (18):3710-5. https://doi.org/10.1093/bioinformatics/bth456.
**7.** Yu, Guangchuang, et al. 2010. "GOSemSim: An R Package for Measuring Semantic Similarity Among Go Terms and Gene Products." Bioinformatics 26 (7):976-78. https://doi.org/10.1093/bioinformatics/btq064.
**8.** Carvalho, B.; et al. Exploration, Normalization, and Genotype Calls of High Density Oligonucleotide SNP Array Data. Biostatistics, 2006.
**9.** Davis S, Meltzer P (2007). "GEOquery: a bridge between the Gene Expression Omnibus (GEO) and BioConductor." Bioinformatics, 14, 1846-1847.
**10.** Du, P., Kibbe, W.A. and Lin, S.M., (2008) 'lumi: a pipeline for processing Illumina microarray', Bioinformatics 24(13):1547-1548.
**11.** Lin, S.M., Du, P., Kibbe, W.A., (2008) 'Model-based Variance-stabilizing Transformation for Illumina Microarray Data', Nucleic Acids Res. 36, e11
**12.** Ritchie, ME, et al. (2015). limma powers differential expression analyses for RNA-sequencing and microarray studies. Nucleic Acids Research 43(7), e47.
**13.** Robinson, MD, McCarthy, DJ, Smyth, GK (2010). edgeR: a Bioconductor package for differential expression analysis of digital gene expression data. Bioinformatics 26, 139-140.
**14.** Colaprico A, wt al. (2015). "TCGAbiolinks: An R/Bioconductor package for integrative analysis of TCGA data." Nucleic Acids Research. doi: 10.1093/nar/gkv1507, http://doi.org/10.1093/nar/gkv1507.
**15.** Robinson, MD, and Oshlack, A (2010). A scaling normalization method for differential expression analysis of RNA-seq data. Genome Biology11, R25.
**16.** Law, CW, Chen, Y, Shi, W, and Smyth, GK (2014). Voom: precision weights unlock linear model analysis tools for RNA-seq read counts. Genome Biology 15, R29.
**17.** Phipson, B, et al. (2016). Robust hyperparameter estimation protects against hypervariable genes and improves power to detect differential expression. Annals of Applied Statistics 10(2), 946-963.
**18.** Wu, D., et al. (2010). ROAST: rotation gene set tests for complex microarray experiments. Bioinformatics 26, 2176-2182.
**19.** Hänzelmann S, Castelo R, Guinney J (2013). "GSVA: gene set variation analysis for microarray and RNA-Seq data." BMC Bioinformatics, 14, 7. doi: 10.1186/1471-2105-14-7, http://www.biomedcentral.com/1471-2105/14/7.
**20.** Langfelder P, Horvath S (2008) WGCNA: an R package for weighted correlation network analysis. BMC Bioinformatics 2008, 9:559.
**21.** Goeman, J. J., van de Geer, S. A., de Kort, F., and van Houwelingen, J. C. (2004). A global test for groups of genes: testing association with a clinical outcome. Bioinformatics, 20(1):93-99.
**22.** Goeman, J. J., van de Geer, S. A., and van Houwelingen, J. C. (2006). Testing against a high-dimensional alternative. Journal of the Royal Statistical Society Series B Statistical Methodology, 68(3):477-493.
**23.** Goeman, J. and Finos, L. (2012). The inheritance procedure: multiple testing of tree-structured hypotheses. Statistical Applications in Genetics and Molecular Biology, 11(1):1-18.
**24.** Monti, S., et al. (2003) Consensus Clustering: A Resampling-Based Method for Class Discovery and Visualization of Gene Expression Microarray Data. Machine Learning, 52, 91-118.
**25.** Wilkerson, D. M, Hayes, Neil D (2010). "ConsensusClusterPlus: a class discovery tool with confidence assessments and item tracking." Bioinformatics, 26(12), 1572-1573.
**26.** Gu Z, Eils R, Schlesner M (2016). "Complex heatmaps reveal patterns and correlations in multidimensional genomic data." Bioinformatics*.*
**27.** Gu, Z., et al. 2014. circlize Implements and enhances circular visualization in R. Bioinformatics, 30(19), pp.2811-2812*.*

## Claims

1. A method for determining AHR activation signature for a biological sample, comprising detecting at least one biological state of at least one AHR biomarker according to table 1 for said sample,
identifying a change of said biological state of said at least one AHR biomarker compared to a house keeping gene or control biomarker, and
assigning said at least one AHR biomarker to said AHR activation signature for said biological sample, if said at least one biomarker provides a significance of said AHR activation signature of p < 0.05 at a minimal number of markers in the signature and/or a fold of change of said AHR activation signature of at least about 1.5 at a minimal number of markers in the signature in the case of up-regulation or of at least about 0.67 at a minimal number of markers in the signature in the case of down-regulation.

2. The method according to claim 1, wherein said biological sample is selected from a sample comprising biological fluids comprising biomarkers, human cells, tissues, whole blood, cell lines, primary cells, IPCs, hybridomas, recombinant cells, stem cells, and cancer cells, bone cells, cartilage cells, nerve cells, glial cells, epithelial cells, skin cells, scalp cells, lung cells, mucosal cells, muscle cells, skeletal muscles cells, straited muscle cells, smooth muscle cells, heart cells, secretory cells, adipose cells, blood cells, erythrocytes, basophils, eosinophils, monocytes, lymphocytes, T-cells, B-cells, neutrophils, NK cells, regulatory T-cells, dendritic cells, Th17 cells, Th1 cells, Th2 cells, myeloid cells, macrophages, monocyte derived stromal cells, bone marrow cells, spleen cells, thymus cells, pancreatic cells, oocytes, sperm, kidney cells, fibroblasts, intestinal cells, cells of the female or male reproductive tracts, prostate cells, bladder cells, eye cells, corneal cells, retinal cells, sensory cells, keratinocytes, hepatic cells, brain cells, kidney cells, and colon cells, and the transformed counterparts of said cell types thereof.

3. The method according to claim 1 or 2, wherein said biological state as detected is selected from mutations, nucleic acid methylation, copy numbers, expression, amount of protein, metabolite, and activity of said at least one AHR biomarker.

4. The method according to any one of claims 1 to 3, wherein said AHR activation signature provides a significance of p < 0.05, preferably of p < 0.01, and more preferably of p < 0.001, and more preferably p < 0.0001 or at least an absolute fold of change of said AHR activation signature of at least about 1.5 in case of up-regulation or at least an absolute fold change of at least about 0.67 in the case of down regulation at a minimal number of markers in the signature

5. The method according to any one of claims 1 to 4, wherein said AHR activation signature comprises about 5, about 10, about 20, about 30 of said AHR biomarkers according to table 1 or at least 10%, at least 20%, at least 30%, at least 40%, at least 50%, at least 60%, at least 70%, at least 80% or at least 90% or more or all of said AHR biomarkers according to table 1.

6. The method according to any one of claims 1 to 5, wherein said AHR activation signature is identified in a sample under physiological conditions or under disease conditions, for example, in biological safety screenings, toxicology studies, cancer, autoimmune disorders, degeneration, inflammation and infection, or under stress conditions, for example, biological, mechanical and environmental stresses.

7. The method according to any one of claims 1 to 6, wherein said method further comprises the step of using said AHR activation signature for unsupervised clustering or supervised classification of said samples into AHR activation subgroups.

8. The method according to any one of claims 1 to 7, wherein said AHR activation signature or AHR activation subgroups are further used to define AHR activation modulated functions, for example, angiogenesis, drug metabolism, external stress response, hemopoiesis, lipid metabolism, cell motility, and immune modulation.

9. A method for monitoring AHR activation in a biological sample in response to at least one compound, comprising performing the method according to any one of claims 1 to 8 on samples that have been obtained during the course of contacting said sample with at least one pharmaceutically active compound, toxin or other modulator compound, wherein said modulator is preferably selected from an inhibitor or an agonist of said biological state.

10. A method for screening for a modulator of AHR activation genes, comprising performing the method according to any one of claims 1 to 8, and further comprising contacting at least one candidate modulator compound with said biological sample or modulating the levels of at least one candidate modulator with said biological sample, wherein a change in the biological state of said at least one AHR biomarker of said signature in the presence of said at least one compound compared to the absence of said at least compound identifies a modulator, wherein said modulator is preferably selected from an inhibitor or an agonist of said biological state.

11. The method according to any one of claims 9 to 10, wherein said modulator is selected from TCDD, FICZ, Kyn, SRI, CH223191, a proteinaceous AHR binding domain, a small molecule, a peptide, a mutated version of a protein, for example an intracellular or recombinantly introduced protein, and a library of said compounds, antibodies, environmental substances, probiotics, toxins, aerosols, medicines, nutrients, galenic compositions, plant extracts, volatile compounds, homeopathic substances, incense, pharmaceutical drugs, vaccines, i.v., compounds or compound mixtures derived from organisms for example animals, plants, fungi, bacteria, archaea, chemical compounds, and compounds used in food or cosmetic industry.

12. The method according to any one of claims 1 to 11, wherein said at least one biological state of said at least one AHR biomarker according to table 1 for said sample is detected using a high-throughput method.

13. A diagnostic kit comprising materials for performing a method according to any one of claims 1 to 12 in one or separate containers, optionally together with auxiliary agents and/or instructions for performing said method.

14. A panel of biomarkers identified by a method according to any one of claims 1 to 8.

15. Use of a panel of biomarkers according to claim 14 for monitoring AHR activation in a biological sample according to claim 9, or for screening for a modulator of AHR activation genes according to any one of claims 10 to 12.


SEQUENCE LISTING

<110>  Deutsches Krebsforschungszentrum (DKFZ)

<120>  AHR signature marker set

<130>  D31660EP

<160>  25

<170>  PatentIn version 3.5

<210>  1
<211>  97
<212>  DNA
<213>  Homo sapiens

<400>  1
tgctgttgac agtgagcgca ggaagaattg ttttaggata tagtgaagcc acagatgtat      60

atcctaaaac aattcttcct ttgcctactg cctcgga                               97


<210>  2
<211>  97
<212>  DNA
<213>  Homo sapiens

<400>  2
tgctgttgac agtgagcgcc ccacaagatg ttattaataa tagtgaagcc acagatgtat      60

tattaataac atcttgtggg atgcctactg cctcgga                               97


<210>  3
<211>  97
<212>  DNA
<213>  Homo sapiens

<400>  3
tgctgttgac agtgagcgaa ggcagaagta tgcaaagcat tagtgaagcc acagatgtaa      60

tgctttgcat acttctgcct gtgcctactg cctcgga                               97


<210>  4
<211>  19
<212>  DNA
<213>  Homo sapiens

<400>  4
gatgggcggc ggaaaatag                                                   19


<210>  5
<211>  21
<212>  DNA
<213>  Homo sapiens

<400>  5
gcgtggattc tgcataatgg t                                                21

<210>  6
<211>  21
<212>  DNA
<213>  Homo sapiens

<400>  6
ctcgccagtg aaatgatggc t                                                21


<210>  7
<211>  21
<212>  DNA
<213>  Homo sapiens

<400>  7
gtcggagatt cgtagctgga t                                                21


<210>  8
<211>  21
<212>  DNA
<213>  Homo sapiens

<400>  8
gacgccttta tcctctctgc g                                                21


<210>  9
<211>  23
<212>  DNA
<213>  Homo sapiens

<400>  9
acgacctgat ccaattctgc cca                                              23


<210>  10
<211>  21
<212>  DNA
<213>  Homo sapiens

<400>  10
ctgcctgggt ttccatcttc t                                                21


<210>  11
<211>  23
<212>  DNA
<213>  Homo sapiens

<400>  11
gccattcatg tcagagctac act                                              23


<210>  12
<211>  22
<212>  DNA
<213>  Homo sapiens

<400>  12
catcaatgac aaggtggcca ag                                               22

<210>  13
<211>  20
<212>  DNA
<213>  Homo sapiens

<400>  13
gggcttgatg gggtgatagg                                                    20


<210>  14
<211>  20
<212>  DNA
<213>  Homo sapiens

<400>  14
acccccatga ctccagagaa                                                    20


<210>  15
<211>  21
<212>  DNA
<213>  Homo sapiens

<400>  15
cttgtgcctg caaaatcgca t                                                  21


<210>  16
<211>  22
<212>  DNA
<213>  Homo sapiens

<400>  16
caccctctag caatgtcaac tc                                                 22


<210>  17
<211>  21
<212>  DNA
<213>  Homo sapiens

<400>  17
cagactcggg atactctctc c                                                  21


<210>  18
<211>  26
<212>  DNA
<213>  Homo sapiens

<400>  18
gctaaccttt gatgctataa ctacga                                             26


<210>  19
<211>  21
<212>  DNA
<213>  Homo sapiens

<400>  19
tttgtgcgca tgtagaatct g                                                  21

<210>  20
<211>  20
<212>  DNA
<213>  Homo sapiens

<400>  20
ccctcctcag gtggtgtttg                                                    20


<210>  21
<211>  20
<212>  DNA
<213>  Homo sapiens

<400>  21
cgacaaatga agcagcgtgt                                                    20


<210>  22
<211>  21
<212>  DNA
<213>  Homo sapiens

<400>  22
ttccacgata tggatttacg g                                                  21


<210>  23
<211>  21
<212>  DNA
<213>  Homo sapiens

<400>  23
gtttcctgtt gcattgagtc c                                                  21


<210>  24
<211>  21
<212>  DNA
<213>  Homo sapiens

<400>  24
ctgaccgcag agtcttttcc t                                                  21


<210>  25
<211>  20
<212>  DNA
<213>  Homo sapiens

<400>  25
gagtggtttg gctggggtaa                                                    20